# EUROPÄISCHE PATENTSCHRIFT

(11) **EP 1 865 827 B1**
(45) Veröffentlichungstag und Bekanntmachung des Hinweises auf die Patenterteilung: **31.10.2012**
(21) Anmeldenummer: 06707640.6
(22) Anmeldetag: 23.03.2006
(51) Int. Cl.: A61B 3/103, A61F 9/01, G02C 7/04

(54) **VERFAHREN UND VORRICHTUNG ZUR ERHÖHUNG DER TIEFENSCHÄRFE EINES OPTISCHEN SYSTEMS**
METHOD AND DEVICE FOR INCREASING AN OPTICAL SYSTEM FOCAL DEPTH
PROCEDE ET DISPOSITIF POUR AUGMENTER LA PROFONDEUR DE CHAMP D'UN SYSTEME OPTIQUE

(30) Priorität: 23.03.2005 DE 102005013558
(43) Veröffentlichungstag der Anmeldung: 19.12.2007
(73) Patentinhaber: Carl Zeiss Meditec AG, 07745 Jena (DE)
(72) Erfinder: SCHRÖDER, Eckhard, 90542 Eckental (DE); WOTTKE, Matthias, 90559 Burgthann (DE); VON BÜNAU, Rudolf, 07749 Jena (DE)
(74) Vertreter: DTS München
(86) Internationale Anmeldenummer: PCT/EP2006/002692
(87) Internationale Veröffentlichungsnummer: WO 2006/100086

(56) Entgegenhaltungen:
- WO-A-2004/052253
- WO-A-2006/056847
- US-A1- 2004 169 820

## Beschreibung

### Gebiet der Erfindung

Die Erfindung betrifft ein Verfahren zur Änderung der Eigenschaften eines optischen Systems mittels kontinuierlichmultifokalem Profil, wobei das Profil eine Komponente zur Erhöhung der Tiefenschärfe des optischen Systems umfasst und die Komponente zur Erhöhung der Tiefenschärfe mindestens aus einem Zernike-Polynom vierter Ordnung berechnet wird.

### Stand der Technik

Die WO 2004/052253 A1 beschreibt eine Excimerlasereinheit und ein entsprechendes Steuerungsverfahren zur Durchführung eines Hornhautabtrages, um Alterssichtigkeit oder auch Presbyopie zu verringern. Dabei wird die Excimerlasereinheit derart gesteuert, dass durch den Abtrag der Hornhaut in dieser eine leicht positive sphärische Aberration der vierten Ordnung (Z(4,0)) erzeugt wird. Durch die so erzeugte Erhöhung in der sphärischen Aberration des Auges wird zumindest teilweise der durch Alterssichtigkeit bedingte Verlust der Akkommodationsfähigkeit des Auges kompensiert. Durch die Einbringung einer positiven sphärischen Aberration der vierten Ordnung kann bei Presbyopie insbesondere eine erhebliche Verbesserung beim Nahvisus erreicht werden.

Es hat sich jedoch herausgestellt, dass durch die Einbringung einer sphärischen Aberration, wie sie in der WO 2004/052253 A1 beschrieben ist, die Basisrefraktion des Auges verändert wird. Dies hat eine Verschlechterung des Fernvisus oder auch der Fernsicht zur Folge. Demnach wird bei dem bekannten Verfahren durch die Einbringung einer Aberration der vierten Ordnung zwar bei Alterssichtigkeit eine Verbesserung des Nahvisus erzielt, dies wird jedoch mit einer Verschlechterung des Fernvisus "erkauft".

### Aufgabe der Erfindung

Es ist folglich eine Aufgabe der vorliegenden Erfindung, ein Verfahren und eine dazugehörige Vorrichtung vorzuschlagen, mit welchen eine Erhöhung der Tiefenschärfe eines optischen Systems erreicht werden kann, die zu einer Verbesserung des Nahvisus ohne entsprechende Verschlechterung des Fernvisus führt. Es soll eine Erhöhung der Tiefenschärfe erreicht werden, die refraktionsneutral für den Fernvisus ist.

### Beschreibung der Erfindung

Die genannte Aufgabe wird erfindungsgemäß durch ein Verfahren der eingangs genannten Art gelöst, welches dadurch gekennzeichnet ist, dass die Komponente zur Erhöhung der Tiefenschärfe zusätzlich aus einem Zernike-Polynom zweiter Ordnung berechnet wird.

Das erfindungsgemäße optische System umfasst vorzugsweise ein Auge. In diesem Fall kann das kontinuierlich-multifokale Profil bevorzugt als Abtrag in der Kontur von Kontaktlinsen oder Intraokularlinsen erfolgen. Bei Intraokularlinsen ist statt Konturänderung auch eine Änderung des Volumen-Brechungsindexes denkbar, die zu entsprechenden Änderungen der Abbildungseigenschaften führen kann. Diese Änderung des Volumen-Brechungsindexes ist besonders bevorzugt über die Linse hin unterschiedlich durchgeführt, so dass die Linse Bereiche mit voneinander abweichenden und vorbestimmten Volumen-Brechungsindices aufweist. Auf diese Weise wird die Brechzahl der Linse und nicht die Form gezielt verändert. Wenn das optische System eine Kontaktlinse oder eine Intraokularlinse umfasst, wird also eine künstliche Linse bearbeitet, und nicht das Auge selber.

Der Abtrag der Oberfläche des Ablationsobjektes, d.h., der Kontaktlinse oder der Intraokularlinse bzw. die Änderung des Brechungsindex erfolgt gemäß einem berechneten Profil. Dieses Profil stellt dar, an welchen Stellen und auf welche Weise die Oberfläche des Ablationsobjektes abgetragen wird bzw. der Brechungsindex geändert wird. Erfindungsgemäß kann das Profil durch eine Summe von Zernike-Polynomen dargestellt werden. Dabei bilden verschiedene Bestandteile der Summe, d.h. verschiedene Zernike-Polynome oder Kombinationen von Zernike-Polynomen verschiedene Komponenten des Abtragsprofils. So können mit verschiedenen in der Summe befindlichen Zernike-Polynomen unterschiedliche Korrekturarten des optischen Systems erreicht werden.

Erfindungsgemäß umfasst das Profil mindestens eine Komponente zur Verbesserung der Tiefenschärfe des optischen Systems. Die Tiefenschärfe kann auch als Schärfentiefe oder Abbildungstiefe bezeichnet werden. Dabei handelt es sich um die Ausdehnung des scharf abgebildeten Bereichs in Richtung der optischen Achse des optischen Systems. Bei einem Auge entspricht die Schärfentiefe demjenigen Distanzbereich, innerhalb welchem das Auge bei einem gegebenen Fokus "scharf sehen" kann. Mit einer Erhöhung der Tiefenschärfe kann der Presbyopie oder auch Alterssichtigkeit entgegengewirkt werden, welche sich dadurch auszeichnet, dass sich die Akkommodationsfähigkeit des Auges verringert.

In der Tat nimmt bei der Altersichtigkeit insbesondere die Fähigkeit der Nahakkommodation ab. Dies bedeutet, dass ein altersichtiges Auge nicht mehr in der Lage ist, auf kurze Distanzen scharf zu sehen. Ab einer bestimmten Nähe ist ein Fokussieren nicht mehr möglich, so dass die entsprechend nah liegenden Objekte (z.B. der Text beim Lesen einer Zeitung) unscharf sind. Wenn nun durch das erfindungsgemäße Verfahren die Tiefenschärfe des Auges in diesem Nahbereich insbesondere in Richtung des Auges verbessert wurde, können nahe Gegenstande weiterhin scharf wahrgenommen werden, obwohl das Auge seine Fähigkeit verloren hat, auf einen derart kurze Distanz zu fokussieren. Obwohl der Gegenstand "zu nahe" am Auge liegt und damit nicht mehr richtig fokussiert wird, wird er dennoch scharf wahrgenommen, da er innerhalb des mittels des erfindungsgemäßen Verfahrens erweiterten Tiefenschärfebereichs liegt.

Die erfindungsgemäße Komponente des Profils zur Erhöhung der Tiefenschärfe wird aus einem Zernike-Polynom vierter Ordnung und zusätzlich aus einem Zernike-Polynom zweiter Ordnung gebildet. Im Unterschied zu bekannten Verfahren sieht die Erfindung demnach zur Erhöhung der Tiefenschärfe nicht nur ein Zernike-Polynom der vierten Ordnung vor, sondern zusätzlich auch einen Zernike-Polynom zweiter Ordnung. Durch die Hinzufügung eines Polynoms zweiter Ordnung wird die Veränderung der Basisrefraktion des bevorzugt Ablationsobjektes, welche sich nachteilig aus der Hinzufügung eines Zernike-Polynoms der vierten Ordnung ergibt, exakt ausgeglichen. Mit der erfindungsgemäßen Addition eines zusätzlichen Terms zweiter Ordnung werden somit die unerwünschten Nebeneffekte, welche sich aus der Einbeziehung eines Terms der vierten Ordnung ergeben, ausgeschaltet. So kann bei Alterssichtigkeit eine Verbesserung des Nahvisus erreicht werden, ohne dass der Fernvisus darunter leidet. Man könnte den erfindungsgemäß hinzugefügten Term zweiter Ordnung also auch als Korrektur-Term bezeichnen, der eine refraktionsneutrale Kompensation erzeugt.

Beim eingangs erwähnten Stand der Technik erfolgt die Einbeziehung eines Zernike-Polynoms zweiter Ordnung bestenfalls in hinlänglich bekannter Weise zum Ausgleich von Kurz- oder Fernsichtigkeit (Myopie oder Hyperopie). Ein zusätzlicher Term zweiter Ordnung als Teil einer Maßnahme zur Verringerung der Alterssichtigkeit (bei Korrektur über die Einbeziehung eines Zernike-Polynoms vierter Ordnung) ist im Gegensatz zur Erfindung aber nicht vorgesehen.

Besonders bevorzugt wird das Profil durch einen Abtrag erzeugt. Ein derartiges Abtragsprofil ist in der Ophthalmologie bekannt, um durch Abtrag von Material auf den Oberflächen eine Linse eines optischen Systems bzw. einem Auge entsprechende kontinuierlich-multifokale Profile zu erzeugen und dadurch eine Änderung der Brechungseigenschaften zu bewirken. Neben einem Abtrag von Material ist es auch denkbar, stellenweise Material hinzuzufügen. Auf diese Weise können die verschiedensten optischen Elemente in einem optischen System mit einem entsprechenden Profil versehen werden.

Weiterhin bevorzugt ist es, das Profil durch eine Änderung der Brechzahl des optischen Systems, insbesondere einer Linse, zu erzeugen. Auf diese Weise ist es möglich, durch gezielte Änderung der Brechkraft, beispielsweise durch Bestrahlung von Material, eine Änderung der refraktiven Eigenschaften des optischen Systems herbeizufügen, ohne dass das Objekt selbst in seinen Ausmaßen geändert wird, wie bei das bei einem Abtrag oder einem Zufügen von Material der Fall ist. Ganz besonders bevorzugt ist die Erzeugung eines Profils durch eine Kombination von Abtrag einerseits und Änderung der Brechzahl des optischen Systems durch Bestrahlung und Änderung der Materialeigenschaften andererseits.

Um eine noch genauere Verbesserung der Tiefenschärfe zu erreichen, kann die Komponente zur Erhöhung der Tiefenschärfe zusätzlich auch einen Zernike-Polynom sechster und/oder höherer Ordnung aufweisen. Durch die Hinzufügung weiterer Polynome höherer Ordnung kann das Profil zur Erhöhung der Tiefenschärfe weiter optimiert werden. Dabei ist zu berücksichtigen, dass, ebenso wie bei der Einführung eines Polynoms vierter Ordnung, die Einführung von Polynomen sechster und/oder höherer Ordnung dazu führen kann, dass unerwünschte Nebeneffekte auftreten. In einer bevorzugten Ausführungsform werden diese dann durch die Hinzufügung zusätzlicher Terme geringerer Ordnung, ausgeglichen.

Es ist auch denkbar, dass zur Erzeugung einer Multifokalität bzw. zur Verbesserung der Tiefenschärfe noch andere Zernike-Polynome, wie bspw. Koma-Polynome (dritte Ordnung) herangezogen werden.

Vorzugsweise ist die Komponente zur Erhöhung der Tiefenschärfe rotationssymmetrisch. Dies bedeutet, dass die Polynome, aus welchen die Komponente zusammengesetzt ist, radiale Polynome sind. Radiale Polynome sind solche Polynome, welche sich in polarer Schreibweise dadurch auszeichnen, dass sie keine Winkelabhängigkeit aufweisen. Besonders bevorzugt wird dabei für den Zernike-Polynom zweiter Ordnung ein Z(2,0)-Term verwendet, für den Zernike-Polynom vierter Ordnung ein Z(4,0)-Term und für den Zernike-Polynom sechster Ordnung ein Z(6,0)-Term.

Wenn es sich bei dem optischen System um ein Auge handelt, kann weiterhin für die Berechnung der Komponente zur Erhöhung der Tiefenschärfe der photopische und der mesopische Durchmesser der Augenpupille berücksichtigt werden. Bei dem photopischen Durchmesser handelt es sich um den Durchmesser der Pupille bei Tageslicht, wohingegen es sich bei dem mesopischen Durchmesser um den Durchmesser der Pupille bei Dämmerung handelt. Durch die Berücksichtigung der Tatsache, dass bei unterschiedlichen Lichtverhältnissen der Durchmesser der Augenpupille variiert, kann eine bessere Optimierung des Profils erfolgen. Wenn bei einem Abtrag ein derart optimiertes Profil verwendet wird, ist die Korrektur der Alterssichtigkeit nicht nur lediglich für eine definierte Pupille (hell oder dunkel) gegeben, sondern die Korrektur ist sowohl bei guten als auch bei schlechten Lichtverhältnissen optimal.

Wenn eine Berücksichtigung der unterschiedlichen Pupillendurchmesser vorgesehen ist, wird vorzugsweise die Ausdehnung des Profils durch den mesopischen Pupillendurchmesser des Auges bestimmt. Der Durchmesser der Pupille bei mesopischen Lichtverhältnissen bildet also die Grundlage für die Festsetzung der optischen Zone der Behandlung. Dies kann insbesondere bedeuten, dass der Durchmesser des Profils im Wesentlichen dem ermittelten mesopischen Pupillendurchmesser entspricht.

Wenn das optische System ein Auge ist und bei der Ermittlung des Abtragsprofils der photopische und der mesopische Durchmesser der Augenpupille berücksichtigt werden, kann diese Berücksichtigung insbesondere dadurch erfolgen, dass das Abtragsprofil in einem zentralen Bereich des optischen Systems innerhalb des photopischen Durchmessers eine stärkere Brechkraft bewirkt als in einem peripheren, ringförmigen Bereich zwischen photopischem und mesopischem Durchmesser. Das Abtragsprofil erzeugt dann bei Verwendung im Zentrum des Ablationsobjektes innerhalb des photopischen Durchmessers eine größere Refraktionskraft als im Bereich um das Zentrum herum bis zum mesopischen Durchmesser. Durch die induzierte stärkere Brechkraft wird der zentrale Bereich insbesondere auch für das Nahsehen optimiert, während der periphere Bereich durch die induzierte etwas schwächere Brechkraft für den Fernvisus optimiert wird.

Besonders bevorzugt wird das Profil an dessen Rand noch durch Übergangszonen ergänzt. Diese Übergangszonen oder Transitionszonen befinden sich um die effektive optische Zone herum, wobei der Durchmesser der effektiven optischen Zone wie bereits beschrieben bevorzugt dem Durchmesser der mesopischen Pupille entspricht. Durch die Hinzufügung von Übergangszonen ist bei der durch die Anwendung des Profils verursachten Profiländerung an deren Rändern ein gleichmäßiger Übergang sichergestellt. So werden stärkere, abrupte und damit unerwünschte Winkeländerungen in der abgetragenen Oberfläche des Ablationsobjektes vermieden.

Es kann weiterhin vorgesehen sein, dass die Koeffizienten der Zernike-Polynome zur Berechnung der Komponente zur Erhöhung der Tiefenschärfe so gewählt sind, dass das Encircled-Energy-Kriterium über einen ausgedehnten Tiefenbereich optimiert wird. Dabei ist es insbesondere von Vorteil, wenn der Durchmesser für das Encircled-Energy-Kriterium dem effektiven Detektordurchmesser des optischen Systems entspricht. Bei dem genannten Detektor handelt es sich bevorzugt um eine auf der Netzhaut befindliche Sehzelle.

Dies bedeutet, dass die Koeffizienten der Polynome so gewählt werden, dass nach Anwendung des Profils auf das Ablationsobjekt das optische System so verändert wurde, dass über einen ausgedehnten Tiefenbereich die Lichtstrahlen von einem durch das optische System zu erfassenden Objekt stets zu einem hohen Prozentsatz innerhalb des effektiven Detektordurchmessers auf der Abbildungsebene des optischen Systems fokussiert werden. Wenn das optische System also ein Auge umfasst, werden die Koeffizienten der Polynome so gewählt, dass bei Anwendung des resultierenden Abtragsprofils in dem Bereich vom Nahvisus zum Fernvisus des Auges von dem Auge erfasste Objekte stets so genau auf der Netzhaut des Auges (beim Auge ist die Abbildungsebene die Netzhaut) fokussiert werden, dass sich auf der Netzhaut für jeden Punkt des erfassten Objekts eine Abbildung ergibt, bei der beispielsweise mindestens 90 % der entsprechenden Lichtstrahlen innerhalb eines Kreises auf der Netzhaut fokussiert werden, dessen Durchmesser nicht größer ist als derjenige einer Sehzelle auf der Netzhaut.

Wenn also die Koeffizienten derart gewählt werden, dass "nur" das Encircled-Energy-Kriterium erfüllt wird, wird nach erfolgtem Abtrag durch das optische System keine exakte Punktfokussierung über den Tiefenbereich erreicht. Dies ist jedoch nicht von Nachteil, solange die Unschärfe nicht größer als der Durchmesser einer Sehzelle ist. Eine Fokussierung, welche exakter als der Durchmesser einer Sehzelle ist, kann in der Tat durch die Sehzellen der Netzhaut ohnehin nicht erfasst werden. Durch den Rückgriff auf das Encircled-Energy-Kriterium kann das Profil demnach vereinfacht bzw. "gröber" gewählt werden, ohne dass dies die Qualität der vorgenommen Korrektur verringert.

Vorzugsweise umfasst das Profil zusätzlich eine Komponente zur Korrektur von Myopie, Hyperopie und/oder Astigmatismus.

In diesem Fall dient das Abtragsprofil nicht nur zur Erhöhung der Tiefenschärfe des optischen Systems, sondern bewirkt bei Verwendung zusätzlich auch eine Korrektur der eben genannten Fehlsichtigkeiten. Das erfindungsgemäße Verfahren zur Korrektur von Alterssichtigkeit kann also auch in ein Verfahren zur Korrektur anderer Fehlsichtigkeiten eingebunden sein.

Besonders bevorzugt wird auch ein Verfahren vorgesehen, bei dem anstatt oder neben einem Abtrag auch eine Änderung der Brechzahl des optischen Systems, insbesondere einer Linse, vorgesehen ist. Auf diese Weise kann statt einer Konturänderung auch eine Änderung des Volumen-Brechungsindexes durchgeführt werden, der ebenfalls zu Änderungen der Abbildungseigenschaften führt. Bei einer solchen Änderung des Volumen-Brechungsindexes ist dieser ebenfalls bevorzugt kontinuierlich-multivokal ausgebildet. Besonders bevorzugt ist eine Kombination des Abtrags einerseits und der Änderung der Brechzahl andererseits vorgesehen. Besonders bevorzugt wird die Änderung der Brechzahl an einer Intraokularlinse vorgenommen - es ist aber auch denkbar, die Änderung der Brechzahl zusätzlich oder ausschließlich an anderen optischen Elementen durchzuführen, wie beispielsweise einer Kontaktlinse oder keinem Brillenglas.

Schließlich stellt die vorliegende Erfindung außerdem zur Lösung der oben genannten Aufgabe eine Vorrichtung zur Durchführung eines der soeben beschriebenen Verfahren bereit.

Eine solche Vorrichtung umfasst bevorzugt eine Einrichtung zur Erzeugung eines Profils eines optischen Systems, bevorzugt umfassend eine Steuereinrichtung für eine formgebende Einrichtung, bevorzugt einen Laser, insbesondere ein Excimer-Laser oder Femtosekunden-Laser sowie eine Steuereinrichtung zur Applikation beispielsweise eines Laserstrahls oder allgemein Strahlungsstrahls auf das zu bearbeitende Objekt. Darüber hinaus ist bevorzugt eine Rechnereinheit vorgesehen, zur Bestimmung von Zernike-Polynomen zur Erhöhung der Tiefenschärfen. Besonders bevorzugt ist auch ein Aberometer vorgesehen, der den Ist-Zustand des optischen Systems bestimmen kann und der insbesondere bevorzugt online auch den Fortgang der Modifikation des optischen Systems durch Änderung des Profils mitteilen bzw. überprüfen kann.

### Kurzbeschreibung der Figuren

- Fig. 1: zeigt ein Flussdiagramm, welches eine Ausführungsform eines erfindungsgemäßen Verfahrens veranschaulicht;
- Fig. 2: zeigt eine Ausführungsform einer erfindungsgemäßen Vorrichtung zur Durchführung eines erfindungsgemäßen Verfahrens.

### Beschreibung bevorzugter Ausführungsformen

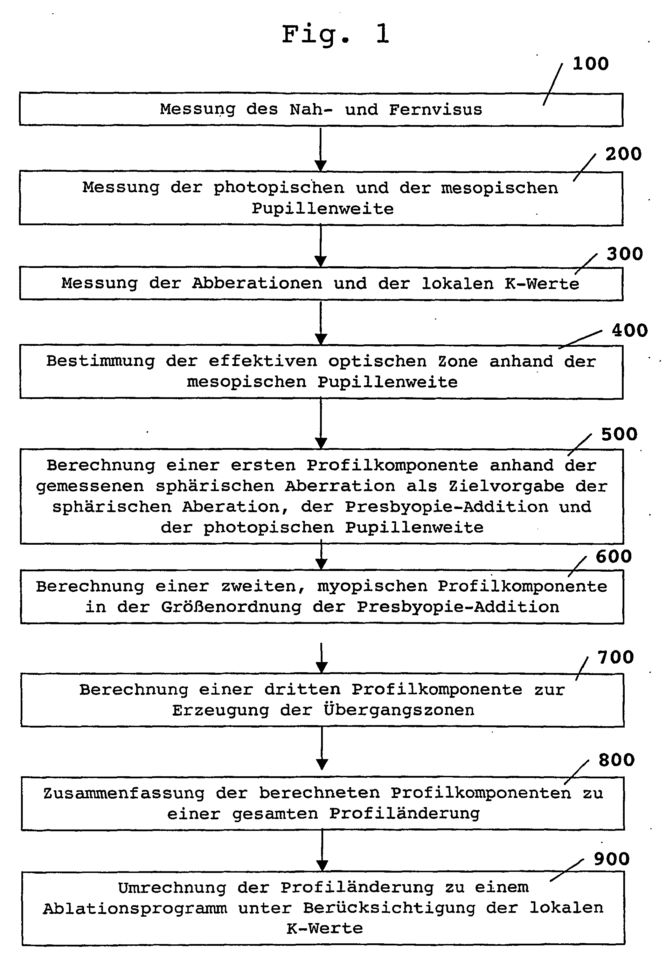

Das erfindungsgemäße Verfahren wird nun nachfolgend mit Bezug auf Fig. 1 erläutert.

Es wird ein Verfahren multifokaler Korrektur von Presbyopie beschrieben. Dieses Verfahren zur Korrektur der Presbyopie kann sowohl bei emmetropen, d.h. bei ausschließlich alterssichtigen, ansonsten aber normalsichtigen Augen durchgeführt werden wie auch bei kurzsichtigen (Myopie), weitsichtigen (Hyperopie) und/oder astigmatischen Augen, die zusätzlich auch alterssichtig sind.

An dieser Stelle ist anzumerken, dass das Flussdiagramm gemäß Fig. 1 ausschließlich das Verfahren bei emmetropen Augen wiedergibt. Bei Augen mit zusätzlicher Fehlsichtigkeit ergeben sich noch weitere Verfahrensschritte.

Wie es Fig. 1 zu entnehmen ist, beginnt das Verfahren damit, dass der Nah- und Fernvisus des zu behandelnden Auges gemessen wird (Schritt 100). Sodann wird die Pupillenweite, d.h. der Durchmesser der Pupille sowohl bei Tageslicht (photopisch) als auch bei Dämmerungsbedingungen (mesopisch) ermittelt (Schritt 200). Daraufhin werden gegebenenfalls die Werte für eine Standardkorrektur (Sphäre, Zylinder, Achse) gemessen, falls entsprechende "Standardfehlsichtigkeiten" (Myopie, Hyperopie, Astigmatismus) vorliegen. Zur Korrektur der Presbyopie werden auch die höheren Aberrationen und die K-Werte gemessen (Schritt 300). Sodann wird aus der ermittelten mesopischen Pupille die optische Zone der Behandlung bestimmt (Schritt 400), wobei es sich bei der optischen Zone um die effektive optische Zone ohne Übergangszone handelt.

Anhand der vorgenommenen Messungen kann nun die zu erfolgende Profiländerung berechnet werden. Zur Korrektur der Alterssichtigkeit wird eine Profiländerung berechnet, die aus zwei Komponenten besteht. Hierbei wird zunächst eine erste Profilkomponente als Zielvorgabe der sphärischen Aberration aus der präoperativen sphärischen Aberration, der Presbyopie-Addition und der Tageslichtpupille berechnet (Schritt 500). Diese erste Komponente zeigt zentral im Bereich der Tageslichtpupille eine höhere Brechkraft auf als in dem Bereich zwischen Tageslichtpupille und mesopischer Pupille. Dabei werden zur Berechnung dieser ersten Komponente die Zernike-Polynome benutzt. Die erste Profilkomponente wird aus dem Z(4,0), dem Z(6,0) und möglicherweise weiteren Zernike-Polynom höherer Ordnung berechnet.

Zusätzlich zur ersten Komponente wird aber auch eine myopische Komponente benötigt, welche die durch die erste Komponente unerwünscht als Nebeneffekt verursachte Refraktionsänderung wieder ausgleicht (Schritt 600). Hierzu wird ein Z(2,0)-Term in der Größenordnung der Presbyopie-Addition verwendet.

Die ermittelte erste und zweite Komponente bilden in ihrer Summe denjenigen Anteil an der Profiländerung, welcher zur Korrektur der Alterssichtigkeit dient.

Weiterhin wird noch eine dritte Komponente für die Profiländerung berechnet (Schritt 700). Diese dritte Komponente sorgt für die Übergangszonen am Rand der Profiländerung zur Vermeidung von stärkeren Winkeländerungen der Hornhaut-Oberfläche.

Schließlich wird gegebenenfalls noch aus den Werten für eine Standardkorrektur eine vierte Komponente zur Korrektur der vorhandenen Myopie, Hyperopie und/oder des Astigmatismus gebildet.

Die berechneten Profilkomponenten werden abschließend zu einer Gesamtprofiländerung zusammengefasst (Schritt 800). Sodann wird diese Gesamtprofiländerung, bestehend aus Z(2,0), Z(4,0), Z(6,0), Übergangszone und gegebenenfalls Standardkorrektur in ein Ablationsprogramm für einen ablativ wirkenden Excimer-Laser umgerechnet (Schritt 900). Eine derartige Gesamtprofiländerung ist aber nur möglich, wenn der Berechnungs- bzw. Behandlungsdurchmesser für alle Komponenten gleich gewählt wird. Es ist aber auch denkbar, auf die Bildung einer Gesamtprofiländerung zu verzichten und etwa bei einer zweiteiligen Behandlung zu bleiben. Etwa derart, dass in einem Teil die GrundKorrektur (Myopie, Hyperopie, Astigmatismus) enthalten ist und in einem zweiten Teil mit anderem Durchmesser die Presbyopie-Korrektur.

Gründe für unterschiedliche Durchmesser können z.B. sein, dass die Grund-Korrektur bei starker Fehlsichtigkeit wegen mangelnder Hornhautdicke nur mit kleinerem Durchmesser zu machen ist, als es der Pupillendurchmesser für den Presbyopie-Anteil fordern würde.

Man gewinnt so weitere Varibilität der Oberfläche, die nicht mehr nur durch ZernikePolynome bis zur 4. Ordnung beschrieben werden kann. Bei der Umrechnung in ein Ablationsprogramm wird der lokale K-Wert der Hornhaut berücksichtigt, damit dieses Ablationsprogramm keine unkontrollierten sphärischen Aberrationen induziert.

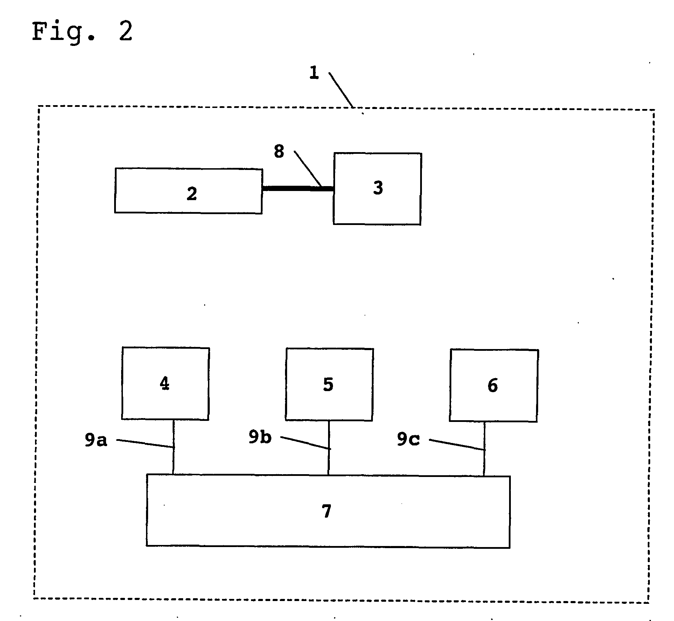

Anhand von Fig. 2 wird nun eine Vorrichtung 1 zur Durchführung des soeben beschriebenen Verfahrens beschrieben.

Die Vorrichtung 1 umfasst eine Licht- oder Strahlquelle 2, eine Strahlenmodifikationseinrichtung 3, eine Wellenfrontanalyseeinrichtung 4, eine Topographieanalyseeinheit 5, ein Mittel 6 zum Messen des photopischen und mesopischen Pupillendurchmessers und ein Mittel 7 zur Ableitung eines Photoablationsprofils.

Bei der Strahlquelle 2 handelt es sich bevorzugt um einen Laser, insbesondere einen refraktiven Laser. In der Regel ist für die Strahlquelle 2 ein Spot-Scanning-Excimerlasersystem vorgesehen. Alternativ kann der Laser auch ein fs-Laser sein. Das Strahlformungs- und Führungssystem 3 dient zur Formung und Ablenkung eines Strahls 8 der Strahlquelle 2. Zur Formung des Strahles 8 verfügt die Einrichtung 3 bevorzugt über Linsensysteme, diffraktive oder refraktive mikrooptische Elemente. Zur Ausrichtung und Ablenkung des Strahls verfügt die Einrichtung 3 bevorzugt über Scanneranordnungen, Prismen oder Spiegel.

Die Wellenfrontanalyseeinrichtung 4 dient zur Analyse der Wellenfront des Strahlengangs im Auge. Die Topographieanalyseeinheit 5 dient zur Analyse der Hornhautoberfläche des Auges. Das Mittel 7 zur Ableitung des Photoablationsprofils, welches auch als Berechnungseinheit bezeichnet werden kann, hat die Aufgabe, aus der von der Wellenfrontanalyseeinrichtung 4, der von der Topographieanalyseeinrichtung 5 gemessene Topographie und der von dem Mittel 6 zum Messen des photopischen und mesopischen Pupillendurchmessers gemessenen Pupillendurchmesser ein entsprechendes Abtragsprofil zu berechnen. Die dafür notwendigen Daten werden der Berechnungseinheit 7 über geeignete Schnittstellen 9a, 9b und 9c von den Messeinheiten 4, 5 und 6 bereitgestellt. Im Übrigen steuert die Berechnungseinheit 7 auch den eigentlichen Abtragsprozess.

Mit dem soeben beschriebenen Verfahren und der dazugehörigen Vorrichtung ergibt sich ein Ablationsprogramm, das zu einer Verbesserung der Tiefenschärfe der optisch wirksamen Zone der Hornhaut und damit zu einer Verbesserung des Nahvisus führt. Im Gegensatz zum Stand der Technik wird dies dergestalt erreicht, dass der Fernvisus nicht beeinträchtigt wird. Der Fernvisus bleibt refraktionsneutral. Da außerdem erfindungsgemäß die Berücksichtigung zweier Pupillengrößen (hell und dunkel) erfolgt, erreicht man eine individuellere Optimierung. Die zusätzliche Einbeziehung von Übergangszonen trägt zur Reduzierung unerwünschter Nebenwirkungen bei, die als "Glare" und "Halo" bezeichnet werden (bei "Glare" oder "Halo" nimmt der Patient nach der Augenoperation ein störendes Scheinen, Blitzen, Glänzen, Strahlen oder auch Funkeln bspw. um Lichtquellen herum war). Der Aufbau definierter Multifokalität über die höhere Ordnung der sphärischen Aberration vermeidet die scharfen Übergänge bei echt bifokalen Zonen-Ansätzen, die sich durch späteren Epithelausgleich zu undefinierter Multifokalität entwickeln.

### Bezugszeichenliste

- 1: Vorrichtung zur Korrektur von Fehlsichtigkeiten
- 2: Lasersystem
- 3: Strahlmodifikationseinrichtung
- 4: Wellenfrontanalyseeinrichtung
- 5: Topographieanalyseeinheit
- 6: Mittel zum Messen des Pupillendurchmessers
- 7: Berechnungseinheit
- 8: Laserstrahl
- 9a - 9c: Schnittstellen
- 100 - 900: Verfahrensschritte

## Patentansprüche

1. verfahren zur Änderung der Eigenschaften eines optischen Systems mittels eines kontinuierlich-multifokalen Profils, das durch Änderung einer Intraokularlinse, einer Kontaktlinse und/oder eines Brillenglases erzeugt wird, wobei
das Profil eine Komponente zur Erhöhung der Tiefenschärfe des optischen Systems umfasst und
die Komponente zur Erhöhung der Tiefenschärfe mindestens aus einem Zernike-Polynom vierter Ordnung berechnet wird,
**dadurch gekennzeichnet, dass** die
Komponente zur Erhöhung der Tiefenschärfe zusätzlich aus einem Zernike-Polynom zweiter Ordnung berechnet wird, wobei
das Zernike-Polynom zweiter Ordnung ein Korrekzur-Term ist, welcher die Basisrefraktion des optischen Systems verändert, die sich nachteilig aus dem Zernike-Polynom der vierten Ordnung ergibt.

2. Verfahren nach Anspruch 1, **dadurch gekennzeichnet, dass** das Profil durch einen Abtrag erzeugt wird.

3. Verfahren nach einem der vorhergehenden Ansprüche, **dadurch gekennzeichnet, dass** das Profil durch eine Änderung der Brechzahl des optischen Systems, insbesondere einer Linse, erzeugt wird.

4. Verfahren nach einem der vorherigen Ansprüche, **dadurch gekennzeichnet, dass** die Komponente zur Erhöhung der Tiefenschärfe zusätzlich aus einem Zernike-Polynom sechster und/oder höherer Ordnung berechnet wird.

5. Verfahren nach einem der vorherigen Ansprüche, **dadurch gekennzeichnet, dass** die Komponente zur Erhöhung der Tiefenschärfe rotationssymetrisch ist.

6. Verfahren nach einem der vorherigen Ansprüche, **dadurch gekennzeichnet, dass** das optische System ein Auge umfasst.

7. Verfahren nach Anspruch 6, **dadurch gekennzeichnet, dass** für die Berechnung der Komponente zur Erhöhung der Tiefenschärfe zusätzlich der photopische und mesopische Durchmesser der Augenpupille berücksichtigt werden.

8. Verfahren nach Anspruch 7, **dadurch gekennzeichnet, dass** der mesopische Pupillendurchmesser die Ausdehnung eines Abtragsprofils bestimmt.

9. Verfahren nach Anspruch 7 oder 8, **dadurch gekennzeichnet, dass** das Abtragsprofil in einem zentralen Bereich des optischen Systems innerhalb des photopischen Durchmessers eine stärkere Brechkraft bewirkt als in einem peripheren ringförmigen Bereich zwischen photopischem und mesopischem Durchmesser.

10. Verfahren nach einem der vorherigen Ansprüche, **dadurch gekennzeichnet, dass** die Koeffizienten der Zernike-Polynome so gewählt sind, dass das Encircled-Energy-Kriterium über einen ausgedehnten Tiefenbereich optimiert wird.

11. Verfahren nach Anspruch 10, **dadurch gekennzeichnet, dass** der Durchmesser für das Encircled Energy-Kriterium dem effektiven Detektordurchmesser des optischen Systems entspricht.

12. Verfahren nach einem der Ansprüche 6 bis 11, **dadurch gekennzeichnet, dass** das Profil zusätzlich eine Komponente zur Korrektur von Myopie, Hyperopie und/oder Astigmatismus umfasst.

13. Vorrichtung zur Korrektur von Fehlsichtigkeiten (1) durch Änderung der Eigenschaften eines optischen Systems mittels eines kontinuierlich-multifokalen Profils, wobei das Profil eine Komponente zur Erhöhung der Tiefenschärfe des optischen Systems umfasst,
umfassend eine Berechnungseinheit (7), die eingerichtet ist, die Komponente zur Erhöhung der Tiefenschärfe aus mindestens einem Zernike-Polynom vierter Ordnung zu berechnen, und vorzugsweise ein Lasersystem (2),
**dadurch gekennzeichnet, dass**
die Berechnungseinheit (7) weiter eingerichtet ist, die Komponente zur Erhöhung der Tiefenschärfe zusätzlich aus einem Zernike-Polynom zweiter Ordnung zu berechnen, wobei
das Zernike-Polynom zweiter Ordnung ein Korrektur-Term ist, welcher die Basisrefraktion des optischen Systems verändert, die sich nachteilig aus dem Zernike-Polynom der vierten Ordnung ergibt.

14. Vorrichtung zur Korrektur von Fehlsichtigkeiten (1) nach Anspruch 13, **dadurch gekennzeichnet, dass** das Profil durch die Vorrichtung zur Korrektur von Fehlsichtigkeiten (1) durch einen Abtrag erzeugbar ist.

15. Vorrichtung zur Korrektur von Fehlsichtigkeiten (1) nach einem der Ansprüche 13 bis 14,
**dadurch gekennzeichnet, dass** durch die Vorrichtung zur Korrektur von Fehlsichtigkeiten (1) das Profil durch eine Änderung der Brechzahl des optischen Systems, insbesondere einer Linse, erzeugbar ist.

16. Vorrichtung zur Korrektur von Fehlsichtigkeiten (1) nach einem der Ansprüche 13 bis 15, **dadurch gekennzeichnet, dass** die Berechnungseinheit (7) eingerichtet ist, die Komponente zur Erhöhung der Tiefenschärfe zusätzlich aus einem Zernike-Polynom sechster und/oder höherer Ordnung zu berechnen.

17. Vorrichtung zur Korrektur von Fehlsichtigkeiten (1) nach einem der Ansprüche 13 bis 16, **dadurch gekennzeichnet, dass** die Komponente zur Erhöhung der Tiefenschärfe rotationssymetrisch ist.

18. Vorrichtung zur Korrektur von Fehlsichtigkeiten (1) nach einem der Ansprüche 13 bis 17, **dadurch gekennzeichnet, dass** das optische System ein Auge, eine Intraokularlinse, eine Kontaktlinse und/oder ein Brillenglas umfasst, welche bearbeitbar sind.

19. Vorrichtung zur Korrektur von Fehlsichtigkeiten (1) nach Anspruch 18, **dadurch gekennzeichnet, dass** die Berechnungseinheit (7) eingerichtet ist, für die Berechnung der Komponente zur Erhöhung der Tiefenschärfe zusätzlich den photopischen und mesopischen Durchmesser der Augenpupille zu berücksichtigen.

20. Vorrichtung zur Korrektur von Fehlsichtigkeiten (1) nach Anspruch 19, **dadurch gekennzeichnet, dass** die Berechnungseinheit (7) eingerichtet ist, den mesopischen Pupillendurchmesser zur Bestimmung der Ausdehnung eines Abtragsprofils zu verwenden.

21. Vorrichtung zur Korrektur von Fehlsichtigkeiten (1) nach Anspruch 19 oder 20, **dadurch gekennzeichnet, dass** die Berechnungseinheit (7) eingerichtet ist, ein Abtragsprofil in einem zentralen Bereich des optischen Systems zu bestimmen, das innerhalb des photopischen Durchmessers eine stärkere Brechkraft bewirkt als in einem peripheren ringförmigen Bereich zwischen photopischem und mesopischem Durchmesser.

22. Vorrichtung zur Korrektur von Fehlsichtigkeiten (1) nach einem der Ansprüche 13 bis 21, **dadurch gekennzeichnet, dass** die Koeffizienten der Zernike-Polynome so gewählt sind, dass das Encircled-Energy-Kriterium über einen ausgedehnten Tiefenbereich optimiert wird.

23. Vorrichtung zur Korrektur von Fehlsichtigkeiten (1) nach Anspruch 22, **dadurch gekennzeichnet, dass** der Durchmesser für das Encircled Energy-Kriterium dem effektiven Detektordurchmesser des optischen Systems entspricht.

24. Vorrichtung zur Korrektur von Fehlsichtigkeiten (1) nach einem der Ansprüche 18 bis 23, **dadurch gekennzeichnet, dass** das Profil zusätzlich eine Komponente zur Korrektur von Myopie, Hyperopie und/oder Astigmatismus umfasst.

## Claims

1. A method of changing the properties of an optical system by means of a continuously multifocal profile which is generated by modification of an intraocular lens, a contact lens and/or a spectacles glass, wherein
the profile comprises a component for increasing the depth of field of the optical system and
the component for increasing the depth of field is calculated at least from a Zernike polynomial of fourth order,
**characterized in that**
the component for increasing the depth of field is additionally calculated from a Zernike polynomial of second order, wherein
the Zernike polynomial of second order is a correction term altering the base refraction of the optical system which adversely results from the Zernike polynomial of fourth order.

2. The method according to claim 1, **characterized in that** the profile is generated by ablation.

3. The method according to any of the preceding claims, **characterized in that** the profile is generated by altering the refractive index of an optical system, in particular of a lens.

4. The method according to any of the preceding claims, **characterized in that** the component for increasing the depth of field is additionally calculated from a Zernike polynomial of sixth and/or higher order.

5. The method according to any of the preceding claims, **characterized in that** the component for increasing the depth of field is rotationally symmetric.

6. The method according to any of the preceding claims, **characterized in that** the optical system comprises an eye.

7. The method according to claim 6, **characterized in that** the photopic and mesopic diameters of the pupil are additionally taken into account for calculating the component for increasing the depth of field.

8. The method according to claim 7, **characterized in that** the extent of an ablation profile is determined by the mesopic diameter of the pupil.

9. The method according to claim 7 or 8, **characterized in that** the ablation profile, in a central area of the optical system within the photopic diameter, results in a higher refractive power than in a peripheral annular area between the photopic diameter and the mesopic diameter.

10. The method according to any of the preceding claims, **characterized in that** the coefficients of the Zernike polynomials are selected such that the encircled-energy-criterion is optimized across an extended depth area.

11. The method according to claim 10, **characterized in that** the diameter for the encircled-energy-criterion corresponds to the effective detector diameter of the optical system.

12. The method according to any of the claims 6 to 11, **characterized in that** the profile additionally comprises a component for correcting myopia, hyperopia and/or astigmatism.

13. A device for correcting defective eyesight (1) by changing the properties of an optical system by means of a continuously multifocal profile, the latter comprising a component for increasing the depth of field of the optical system,
comprising a calculation unit (7) which is configured for calculating the component for increasing the depth of field from at least one Zernike polynomial of fourth order, and preferably a laser system (2),
**characterized in that**
the calculation unit (7) is further configured for calculating the component for increasing the depth of field additionally from a Zernike polynomial of second order, wherein
the Zernike polynomial of second order is a correction term altering the base refraction of the optical system which adversely results from the Zernike polynomial of fourth order.

14. The device according to claim 13 for correcting defective eyesight (1), **characterized in that** the profile can be generated by the device for correcting defective eyesight (1) through ablation.

15. The device according to any of the claims 13 to 14 for correcting defective eyesight (1), **characterized in that** the profile can be generated by the device for correcting defective eyesight (1) through an alteration of the refractive index of the optical system, in particular of a lens.

16. The device according to any of the claims 13 to 15 for correcting defective eyesight (1), **characterized in that** the calculation unit (7) is configured for calculating the component for increasing the depth of field additionally from a Zernike polynomial of sixth and/or higher order.

17. The device according to any of the claims 13 to 16 for correcting defective eyesight (1), **characterized in that** the component for increasing the depth of field is rotationally symmetric.

18. The device according to any of the claims 13 to 17 for correcting defective eyesight (1), **characterized in that** the optical system is an eye, an intraocular lens, a contact lens and/or a spectacles glass, which can be processed.

19. The device according to claim 18 for correcting defective eyesight (1), **characterized in that** the calculation unit (7) is configured for additionally considering the photopic and mesopic diameters of the pupil to calculate the component for increasing the depth of field.

20. The device according to claim 19 for correcting defective eyesight (1), **characterized in that** the calculation unit (7) is configured for using the mesopic diameter of the pupil for determining the extent of an ablation profile.

21. The device according to claim 19 or 20 for correcting defective eyesight (1), **characterized in that** the calculation unit (7) is configured for determining an ablation profile in a central area of the optical system which results in a higher refractive power within the photopic diameter than in a peripheral annular area between the photopic diameter and the mesopic diameter.

22. The device according to any of the claims 13 to 21 for correcting defective eyesight (1), **characterized in that** the coefficients of the Zernike polynomials are selected such that the encircled-energy-criterion is optimized across an extended depth area.

23. The device according to claim 22 for correcting defective eyesight (1), **characterized in that** the diameter for the encircled-energy-criterion corresponds to the effective detector diameter of the optical system.

24. The device according to any of the claims 18 to 23 for correcting defective eyesight (1), **characterized in that** the profile additionally comprises a component for correcting myopia, hyperopia and/or astigmatism.

## Revendications

1. Procédé de modification des propriétés d'un système optique à l'aide d'un profil multifocal en continu produit par modification d'une lentille intraoculaire, d'une lentille de contact et/ou d'un verre de lunettes ;
le profil comprenant une composante d'augmentation de la profondeur de champ du système optique ; et
la composante d'augmentation de la profondeur de champ étant calculée au moins à partir d'un polynôme de Zernike de quatrième ordre ;
**caractérisé en ce que** :
la composante d'augmentation de la profondeur de champ est calculée en outre à partir d'un polynôme de Zernike de deuxième ordre ;
le polynôme de Zernike de deuxième ordre étant un terme correctif modifiant la réfraction de base du système optique produite de façon désavantageuse par le polynôme de Zernike de quatrième ordre.

2. Procédé selon la revendication 1, **caractérisé en ce que** le profil est produit par un retrait.

3. Procédé selon l'une quelconque des revendications précédentes, **caractérisé en ce que** le profil est produit par une modification de la valeur de rupture du système optique, notamment d'une lentille.

4. Procédé selon l'une quelconque des revendications précédentes, **caractérisé en ce que** la composante d'augmentation de la profondeur de champ est en outre calculée à partir d'un polynôme de Zernike de sixième ordre et/ou d'ordre supérieur.

5. Procédé selon l'une quelconque des revendications précédentes, **caractérisé en ce que** la composante d'augmentation de la profondeur de champ est symétrique en rotation.

6. Procédé selon l'une quelconque des revendications précédentes, **caractérisé en ce que** le système optique comprend un oeil.

7. Procédé selon la revendication 6, **caractérisé en ce que** le diamètre photopique et mésopique de la pupille de l'oeil sont en outre pris en compte pour le calcul de la composante d'augmentation de la profondeur de champ.

8. Procédé selon la revendication 7, **caractérisé en ce que** le diamètre de pupille mésopique détermine l'ampleur d'un profil de retrait.

9. Procédé selon la revendication 7 ou 8, **caractérisé en ce que** le profil de retrait engendre une force de rupture plus importante dans une région centrale du système optique située à l'intérieur du diamètre photopique que dans une région périphérique circulaire située entre les diamètres photopique et mésopique.

10. Procédé selon l'une quelconque des revendications précédentes, **caractérisé en ce que** les coefficients des polynômes de Zernike sont choisis de façon à optimiser le critère d'énergie encerclée sur une région de profondeur étendue.

11. Procédé selon la revendication 10, **caractérisé en ce que** le diamètre du critère d'énergie encerclée correspond au diamètre efficace du détecteur du système optique.

12. Procédé selon l'une quelconque des revendications 6 à 11, **caractérisé en ce que** le profil comprend en outre une composante de correction de la myopie, de l'hyperopie et/ou de l'astigmatisme.

13. Dispositif de correction d'amétropies (1) par modification des propriétés d'un système optique à l'aide d'un profil multifocal en continu, le profil comprenant une composante d'augmentation de la profondeur de champ du système optique ;
comprenant une unité de calcul (7) conçue pour calculer la composante d'augmentation de la profondeur de champ à partir d'au moins un polynôme de Zernike de quatrième ordre ainsi que de préférence un système laser (2) ;
**caractérisé en ce que** :
l'unité de calcul (7) est en outre conçue pour calculer la composante d'augmentation de la profondeur de champ à partir d'un polynôme de Zernike de deuxième ordre ;
le polynôme de Zernike de deuxième ordre étant un terme correctif modifiant la réfraction de base du système optique résultant de façon désavantageuse du polynôme de Zernike de quatrième ordre.

14. Dispositif de correction d'amétropies (1) selon la revendication 13, **caractérisé en ce que** le profil peut être réalisé par un retrait réalisé par le biais du dispositif de correction d'amétropies (1).

15. Dispositif de correction d'amétropies (1) selon l'une quelconque des revendications 13 à 14, **caractérisé en ce que** le profil peut être réalisé par une modification de la valeur de rupture du système optique, notamment d'une lentille, par le biais du dispositif de correction d'amétropies (1).

16. Dispositif de correction d'amétropies (1) selon l'une quelconque des revendications 13 à 15, **caractérisé en ce que** l'unité de calcul (7) est en outre conçue pour calculer la composante d'augmentation de la profondeur de champ à partir d'un polynôme de Zernike de sixième ordre et/ou d'ordre supérieur.

17. Dispositif de correction d'amétropies (1) selon l'une quelconque des revendications 13 à 16, **caractérisé en ce que** la composante d'augmentation de la profondeur de champ est symétrique en rotation.

18. Dispositif de correction d'amétropies (1) selon l'une quelconque des revendications 13 à 17, **caractérisé en ce que** le système optique comprend un oeil, une lentille intraoculaire, une lentille de contact et/ou un verre de lunettes pouvant être retravaillés.

19. Dispositif de correction d'amétropies (1) selon la revendication 18, **caractérisé en ce que** l'unité de calcul (7) est en outre conçue pour tenir compte des diamètres photopique et mésopique de la pupille de l'oeil pour le calcul de la composante d'augmentation de la profondeur de champ.

20. Dispositif de correction d'amétropies (1) selon la revendication 19, **caractérisé en ce que** l'unité de calcul (7) est conçue pour utiliser le diamètre de pupille mésopique en vue de déterminer l'ampleur d'un profil de retrait.

21. Dispositif de correction d'amétropies (1) selon la revendication 19 ou 20, **caractérisé en ce que** l'unité de calcul (7) est conçue pour déterminer un profil de retrait dans une région centrale du système optique provoquant l'application d'une force de rupture plus importante à l'intérieur du diamètre photopique que dans une région périphérique circulaire située entre les diamètres photopique et mésopique.

22. Dispositif de correction d'amétropies (1) selon l'une quelconque des revendications 13 à 21, **caractérisé en ce que** les coefficients des polynômes de Zernike sont choisis de façon à optimiser le critère d'énergie encerclée sur une région de profondeur étendue.

23. Dispositif de correction d'amétropies (1) selon la revendication 22, **caractérisé en ce que** le diamètre du critère d'énergie encerclée correspond au diamètre efficace du détecteur du système optique.

24. Dispositif de correction d'amétropies (1) selon l'une quelconque des revendications 18 à 23, **caractérisé en ce que** le profil comprend en outre une composante de correction de la myopie, de l'hyperopie et/ou de l'astigmatisme.
